Europäisches Patentamt

**European Patent Office**

Office européen des brevets

(19)

(11) Publication number: **0 028 209**
A1

(12)

# EUROPEAN PATENT APPLICATION

(21) Application number: **80850149.8**

(22) Date of filing: **13.10.80**

(51) Int. Cl.³: **A 63 B 21/00, A 61 B 5/10**

(30) Priority: **24.10.79 SE 7908789**

(43) Date of publication of application: **06.05.81**
**Bulletin 81/18**

(84) Designated Contracting States: **AT BE CH DE FR GB IT LI LU NL SE**

(71) Applicant: **Smidak, Emil Frank, 14 Chemin Hauts-Crets, CH-1223 Cologny (CH)**

(72) Inventor: **Smidak, Emil Frank, 14 Chemin Hauts-Crets, CH-1223 Cologny (CH)**

(74) Representative: **Lindblom, Erik J, Caroline Östbergs väg 5, S-122 35 Enskede (SE)**

(54) **A device designed to stimulate exercise by the individual.**

(57) A device designed to stimulate exercise by the individual (1), containing a unit (a bicycle ergometer) (2) which is activated by the effort expended by the individual. The load imposed on the unit and thus the degree of effort to be expended by the individual may be varied over a period of time in accordance with a programme. The programming equipment is in the form of a microprocessor. The programming equipment is so arranged that on the one hand it will control the variation in load over a period of time, and that on the other hand it will add (14) oxygen to the supply of air depending on the degree of effort.

A device (12) for detecting the effort expended by the individual is connected to the programming equipment and as soon as the effort exceeds a predetermined value will cause the programming equipment to select a less demanding programme or alternatively to shut down the programme.

EP 0 028 209 A1

- 1 -

TITLE OF THE INVENTION: A device designed to stimulate exercise by the individual.

TECHNICAL FIELD.

The present invention relates to a device designed to stimulate exercise by the individual and in particular to such devices for stimulating exercise which contain a unit activated by the effort expended by the individual.

The unit activated by the effort expended by the individual may, for example, consist of a bicycle ergometer, a rowing machine, a disc-loading bar bell device, a moving belt offering resistance to walking or running and other similar units.

DESCRIPTION OF THE PRIOR ART.

Devices for stimulating exercise are already familiar in a large number of models. The one which is probably used most is the so-called bicycle ergometer. Although other devices are in use and although these may be used very well in conjunction with the present invention, for the sake of simplicity the following description will be illustrated only by means of a bicycle ergometer.

It is normally possible in the case of these devices for stimulating exercise not only to set the operating speed oneself, but also to regulate the load oneself.

The most normal application for exercise-stimulating devices is for them to be used at a predetermined set speed, usually related to a timing device, measuring gauge, dial indicator or similar, and for the load to increase in intensity in relation to the elapsed time, either in accordance with a specific programme which may be set manually or in an arbitrary manner depending on ability.

## DESCRIPTION OF THE PRESENT INVENTION.

### TECHNICAL PROBLEM

It is a known fact that the human body is built for activity and not for resting. Since a considerable number of people now spend their lives sitting, lying down or walking, the natural and highly important stimulation of the tissues and the internal organs through physical work has disappeared. It is also a fact that the majority of the body tissues have the in-built characteristic of being able to adapt to a very high degree to either inactivity or activity. This is especially true of muscle, bone and blood.

It is possible in this way for the physical ability to work to be affected in both an inactive and an active sense, with the result that a need for regular exercise has arisen for the majority of those in sedentary occupations.

What is important is that it should be the individual himself who chooses to expose himself to exertion and the use of an exercise-stimulating device is a simple means of performing this activity.

It was subsequently found to be important for the individual who is taking exercise to have access to fresh, clean air, preferably containing additional oxygen.

To use a technical expression, the volume of blood pumped out by the heart each minute is known as the "minute volume" of the heart, and the quantity displaced by each beat is known as the "stroke volume" of the heart.
This may be used to produce the relationship that the
minute volume is equal to the stroke volume times the
frequency or pulse rate of the heart.

Thus an individual with a small stroke volume must
be allowed to reach a high pulse rate if he is to have a
high minute volume, which means that the pulse rate will
increase as the load increases. The pulse repetition
frequency will thus be an individual characteristic and
will depend, amongst other things, on the physical
condition of the individual.

A recommendation was subsequently made for so-called
interval training, in which the individual is subjected
to relatively hard exertion for a short period, followed
by a period of rest or lighter exertion, and where the
length of the period may be of the order of 1 minute.
Some movement and some imposition of load should still
occur during the rest period, thereby utilizing the
so-called vein pump, i.e. the massaging effect of the
muscles on the blood vessels which stimulates the removal
of metabolic products from the musculature.

This in itself means that there is a need for an
exercise or training programme directly suited to each
person, i.e. a programme tailored to suit the individual,
due to the fact that different individuals are in
different physical condition, different individuals have
a different level of endurance and different individuals
are of different physical build.

There is a need for a set training programme which
may be used over and over again, thereby enabling the
result of the training to be assessed on each occasion.

It is, in fact, of great psychological value to be
able to see improvements in one's physical condition
resulting from the use, and preferably the regular use,
of the exercise-stimulating device.

Once a training programme has been used and has
produced a certain improvement in one's physical condition, then the need will arise to be able to modify the training programme by simple means into a more difficult programme.

In certain circumstances it may be interesting for one individual to train according to a training programme designed for a different individual, usually in such cases a leading individual within one or more branches of sport, thereby being given the opportunity to compare their relative physical condition.

There has always been a problem in producing a device to stimulate exercise by the individual which is of such a nature that it takes account of the above requirements, yet to produce a design which is such that its prime cost is sufficiently low for the device to be a practical proposition for the man in the street.

There is a further problem in producing a training or exercise programme which is tailored to suit the individual and which is of such a nature that it may be reproduced at will with great accuracy.

Yet another problem is encountered in making an acceptably accurate comparison of the result obtained for a given programme on one occasion with the result for the same programme but on a different occasion.

Finally, there is the wish on the one hand to be able to use a training or exercise programme tailored to suit the individual, if so desired, and on the other hand to be able to use one or more predetermined training or exercise programmes, if so desired. It may also be found stimulating to use a training programme specifically designed for one particular sport.

It should be mentioned in this connection that it is often important in the area of rehabilitation to be able on the one hand to modify the training programme rapidly and with little increase in load, and on the other hand to be able to assess the physical condition of the individual to within a small margin of error.

A problem is also associated with the fact that exertion of this kind requires large quantities of oxygen and the breathing becomes heavier in order to meet this need, which usually means that large quantities of polluted air (containing dust particles and gases) must pass through the respiratory system. The problem is how to clean the air in a simple manner, and how to cool the air and/or to add oxygen to the air.

There is also a problem associated with the simple control and regulation of the quantity of air, the temperature of that quantity of air and the addition of oxygen to the air depending on the level of exertion.

Should cooled air be used, then it may be found advisable for this to be used in combination with a source of heat directed at the face of the individual; the most suitable source of heat is infra-red radiation.

It is particularly important in the case of rehabilitation that an indication should be given of the effort being expended by the individual during the training programme and that as soon as the level of exertion exceeds a certain predetermined value, then the training is either terminated or possibly a less demanding training programme selected.


## SOLUTION.

The aim of the present invention is to specify an exercise-stimulating device containing a unit activated by the effort expended by the individual.

The aim of the present invention is to specify a possible means whereby the load imposed on the unit and thus the degree of effort to be expended by the individual may be varied over a period of time. The variation in load shall be controlled by a programme suited to the individual in question allotted by the programming equipment, or the individual may select one or more of a number of pre-programmed programmes. The programming equipment will benefit by being fitted with a microprocessor.

- 6 -

0028209

The invention also specifies a possible means whereby the programming equipment may on the one hand control the variation in load over a period of time, and on the other hand add oxygen to a supply of air directed at the individual. The oxygen may be added in relation to the effort expended.

It is also possible in accordance with the present invention for a device for detecting the effort expended by the individual (a device for monitoring the pulse of the individual) to be connected to the programming equipment and which as soon as the effort exceeds a predetermined value, for example a pulse rate of 180, will cause the programming equipment to select a less demanding programme or to terminate the training entirely.

It is possible in accordance with the present invention to arrange and direct a supply of air towards the face of the individual and for the intensity of and the variation in the supply of air to be controlled to advantage by means of programming equipment. The air supply will benefit if it is first passed through an air cleaning device.

By causing the air supply to be directed towards the face of the individual it is possible, when bicycle ergometers are being used, to create a psychological effect for the individual concerned that he is actually experiencing training under realistic conditions, since the individual will feel the wind in his face.

The present invention specifies a possible means whereby the intensity of the air supply may be varied and will preferably increase in relation to increasing effort.

The invention also specifies a possible means whereby the temperature of the air supply may be regulated to produce either heated or cooled air. Air which has been cooled would be preferable. Where the air supply is at a low temperature, the face may at the same time be exposed to a source of heat, the most suitable source of heat being infra-red radiation.

## ADVANTAGES

The principal advantages of the present invention
are that the device specified in the invention has
succeeded in solving the problems indicated above.

The invention also specifies a possible means
whereby the load may be regulated automatically in
relation to the elapsed time and whereby such measures
may be taken as will preclude any risk of the individual
being over-exerted.

The aim of the invention is also to specify a
programme which is tailored to suit the individual and
which will permit one and the same exercise-stimulating
device to be controlled according to different training
programmes, for instance by means of a programme card
which is introduced into a slot.

Also included amongst the advantages is the fact
that the programme which is tailored to suit the
individual may be programmed-in by the person himself,
and that his physical condition can be assessed rapidly.

Amongst the advantages of the present invention is
the possibility of executing the training programme in
a controlled air supply. This should preferably be
purified of dust particles, toxic gases and gas mixtures.

By causing the air supply to pass through an air
cooling device, a refreshing effect will be produced on
the individual who will then exert himself in accordance
with the training programme, with the result that the
training will become more realistic.

What may be regarded as the principal characteristics
of an exercise-stimulating device in accordance with the
present invention are indicated in the first paragraph
of the following Patent Claim.

DESCRIPTION OF THE DRAWINGS.

A preferred embodiment indicating the significant characteristic features of the present invention is described in greater detail with reference to the attached drawing, in which

Fig. 1    shows a perspective view of an individual using a unit activated by the effort expended by the individual in the form of a bicycle ergometer and with a unit producing a supply of air in the direction of the individual's face attached to the bicycle ergometer and where the unit and the load in relation to the elapsed time are controlled by a microprocessor.

Fig. 2    illustrates with solid lines a suggested first training programme designed to suit the individual, and with dotted lines a second, less demanding training programme designed to suit the individual, for the purpose of thereby demonstrating the functions of the present invention,    and

Fig. 3    shows in the form of a block diagram an embodiment of a control unit which may be used in conjunction with the present invention.

DESCRIPTION OF THE PREFERRED EMBODIMENT.

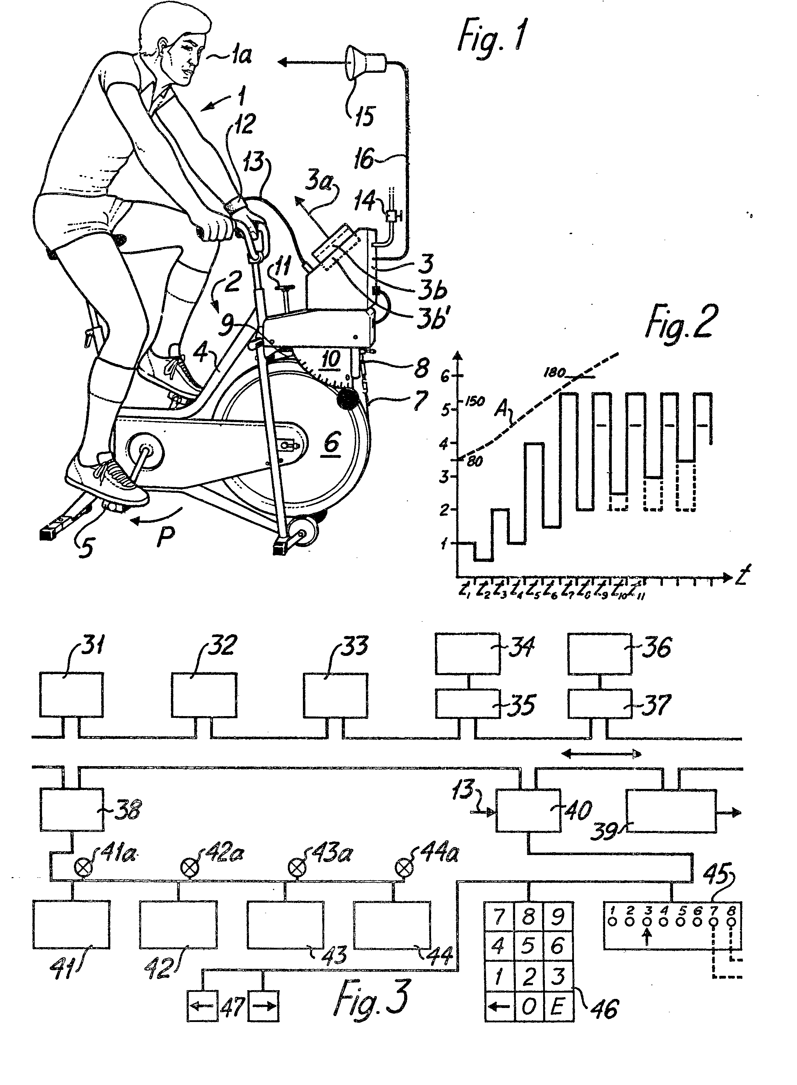

Fig. 1 presents a perspective view of a device to stimulate exercise by the individual in accordance with the present invention. The individual has been identified with the reference designation  1  and is using a unit  2  activated by the effort expended by the individual. This unit is shown in Fig. 1 in the form of a bicycle ergometer, but could of course consist of other devices. Also shown is a unit  3  producing a supply of air in the direction of the individual's face driven by and containing a microprocessor. It may be seen from Fig. 1 that the supply of air 3a is directed towards the face 1a of the individual 1 thereby causing the individual to gain the impression of a wind in his face. The supply of air 3a is arranged so as to pass through a filter 3b , used as an air cleaning device (for removing dust particles and/or toxic gases) and through a temperature regulating circuit 3b' . The air cleaning device and the unit 3 also contain a fan, of which the blades, connected to a driving motor, are controlled via a lead from the microprocessor in such a way that the intensity of the air supply may be varied. It is thus possible, by means of signals transmitted through the lead from the microprocessor, to control the driving motor for the fan so that it will produce an increase in the air supply where there is increased effort on the part of the individual 1 . To the individual's wrist is attached a device 12 for detecting the pulse of the individual. This device 12 is connected by means of a lead 13 to a pulse recording device connected to the microprocessor. Oxygen may be added via the pipe 14 to the air which passes through the unit 3 .

The individual 1 in Fig. 1 is using a bicycle ergometer 2 consisting of a frame 4 to which pedals 5 are attached so as to rotate. The pedals 5 are rotated in the direction of the arrow P and thus drive a wheel 6 . Around the wheel 6 runs a brake band 7 of which one end 8 is firmly attached to the frame 4 whereas a variable tension may be applied to the other end 9 , thus producing different loads for the individual. This variable tension is produced in a unit 10 . This unit may be activated partly by means of a manual activating device 11 , and partly by means of a device controlled by the microprocessor, (this is not shown in Fig. 1), which produces an equivalent effect to that produced by the device 11 .

0028209

A heat lamp 15 is so arranged as to direct a ray of heat at the face of the individual and is controlled by the microprocessor via the lead 16 .

The aim of the present invention is also to cause the load imposed on the unit and thus the degree of effort to be expended by the individual to be variable over a period of time. In order to achieve this a sensing device for measuring the load on the unit will usually be required, but above all a device will be required which will control the intensity of the load imposed over a period of time, which is done by means of the device controlled by the microprocessor.

The variation in load over a period of time is controlled by a programme suited to or selected for the person in question and allotted by the programmimg equipment, i.e. a training or exercise programme tailored to suit the individual. This programme may be entered in the form of a card which is fed into a slot in the programming equipment, thereby causing the microprocessor to generate the number of output signals corresponding to the time sequence which is programmed on the card.

Fig. 2 shows one such training programme tailored to suit the individual, according to which the load imposed on the bicycle ergometer 2 during an initial period of time t1 will exhibit the value 1 , after which the load will fall to the value 0.5 during a second period of time t2 . The load will increase to the value 2 during the next period t3 and will then fall to the value 1 during the following period t4 . The load will thus be increased progressively, but with the provision of intermediate rest periods at reduced load.

The typical layout illustrated in Fig. 2 shows an initial training programme tailored to suit the individual drawn with solid lines, and this programme will produce a load with a maximum value of 5.5 after the time t7 has elapsed, whereas the load imposed during the rest periods is increased by a unit value corresponding to 0.5 between each of the series of imposed loads.

It has also been assumed that the pulse detecting circuit 12 will indicate that the pulse repetition frequency is as shown in the dotted curve A . As the pulse rate reaches a predetermined maximum value of 180, the training programme tailored to suit the individual drawn with a solid line will cease to be switched in after the time t8 has elapsed, and another, less demanding training programme tailored to suit the individual will be switched in; this will in fact be the one drawn with dotted lines which will produce a load corresponding to the value 4.5 and a rest period in which the load has a value equal to 2 .

Training may either be stopped as the indicated value for the pulse repetition frequency is reached or the training programme tailored to suit the individual may be reduced as shown in Fig. 2 and training continued.

The point in time at which the limit value for the pulse repetition frequencies is reached may thus be used as a measure of physical condition, and these timings may be compared from one occasion to the next.

Fig. 3 is in the form of a block diagram illustrating the use of a microprocessor to control the sequence described above.

The typical layout shows a microprocessor 31 (of the type known as 8085) . This processor 31 works in conjunction with a reading memory 32 (EPROM 2716) with a permanently stored programme, a writing/reading memory 33 (RAM2114) with a variable programme, a training programme input and output unit 34 (PERTEC FD 200 Microfloppy) with an associated adapter unit 35 , a plotter 36 with an associated adapter unit 37 and the output channels 38 and 39 and the input channels 40 .

The output channels 38 and 39 work in conjunction with a number of numerical display units 41-44 with associated indicator lamps 41a-44a.

The input channels 40 work in conjunction with a programme switching device 45 , a keyboard 46 and a device 47 for activating jump instructions in the programme.

It is assumed that the manner in which the internal components are connected will be fairly obvious to a specialist when considering the following functional description.

It is presumed that the display unit 41 will indicate the sequence in which the programme is working, i.e. t1 , t2 or other periods of time.

The display unit 42 may indicate the value of the load during the sequence shown on the unit 41 .

The display unit 43 indicates the duration of the periods in the sequence in question.

The display unit 44 indicates the intensity of the fan 3b .

Assuming that use will be made of a programme in accordance with Fig. 2 which has already been entered, then this programme is connected to the switching device 45 and is transmitted via the input channels 40 to the writing/reading memory 33 . The display unit 41 will now indicate the sequence (t1, t2 ...) in which the programme is working, display unit 42 will indicate the value of the load (1, 0.5 ....), display unit 43 will indicate the duration (let us say 60 seconds) of the sequence, and it is suggested that a meter showing the time remaining in the sequence should be installed in a position visible to the individual. The display unit 44 may indicate other information, for example the intensity of the fan.

To the input channels 40 is connected a lead 13 of which the signal is dependent on the pulse rate of the individual. At a value in excess of a predetermined value either the programme will be terminated or a less demanding programme will be selected.

If it is wished to advance the programme or to repeat parts of it, then this may be done by means of the buttons 47 .

One of the indicator lamps 41a-44a will light up if a new programme is to be entered.

By keying in a certain combination of figures via the keyboard 46 it is possible to adjust each and every one of the functions 41-44 and to set them to the programme tailored to suit the individual.

The button "E" is pressed when the programme has been completed, which will cause the selected programme to be entered on a card in the unit 34 .

The programme and the result of the training (variations in pulse rate) may be plotted out on a plotter 36 for statistical purposes.

Although the present invention uses the variation in pulse rate as an indication of the effort expended, there is nothing to prevent this being replaced by or used in conjunction with an assessment of the breathing rate and/or the blood pressure and limit values also being laid down for these parameters above which the training programme would be modified or terminated.

As far as concerns the output channels 39 , these are connected so as to control different functions of the programme.

Thus

a) the load may be adjusted and varied as a function of the time;

b) the fan motor may be adjusted and varied as a function of the time;

c) the addition of oxygen to the air supply may be adjusted and varied as a function of the time;

d) the temperature of the air in the air supply may be adjusted and varied (3b') as a function of the time;

e) the irradiation of the face of the individual with heat may be adjusted and varied as a function of the time.

Although it is usual for the functions to be capable of variation over a period of time, there is

0028209

nothing to prevent them remaining constant over a period
of time.

The intensity of the air supply may of course be
adjusted in line with the level of effort expended so
that an increase in effort will produce an increase in
the speed of the air-flow.

This invention is not of course restricted to the
embodiment shown above as an example, but may be modified
within the context of the following Patent Claim.

Although the present invention has been illustrated
by means of an exercise-stimulating device programmed
with a demanding programme, it should be noted that a
less demanding programme should be used for medical
purposes. Particularly in the latter case it may be
extremely important to add oxygen to the air supply. It
is especially important for the air supply to pass through
a filter in which the sulphur contained in the air is
filtered out or separated.

The invention may be used to advantage for
therapeutic purposes in connection with mental illnesses
and for old people with reduced mobility.

## Claims

1.  A device designed to stimulate exercise by the
individual containing a unit (a bicycle ergometer)
activated by the effort expended by the individual, in
which the load imposed on the unit and thus the degree
of effort to be expended by the individual may be varied
over a period of time in accordance with a programme,
c h a r a c t e r i z e d  in that the variation in load
over a period of time is controlled by a programme
suited to the individual in question and allotted by the
programming equipment, said programming equipment being
in the form of a microprocessor  (31)  working together
with a reading memory  (32)  with a permanently stored
programme, a writing/reading memory  (33)  with a
variable programme and a training programme input and
output unit  (34)  with an associated adapter unit  (35).

2.  A device in accordance with Claim 1, c h a r a c t e r -
i z e d  in that the programming equipment on the one
hand controls the variation in load over a period of
time, and on the other hand adds oxygen to a supply of
air depending on the effort.

3.  A device in accordance with Claim 1, c h a r a c t e r -
i z e d  in that a device for detecting the effort expended
by the individual is connected to the programming
equipment and as soon as the effort exceeds a predetermined
value will cause the programming equipment to select a
less demanding programme or alternatively to shut down
the programme.

4.  A device in accordance with Claim 1, c h a r a c t e r -
i z e d  in that a supply of air is directed at the face
of the individual and is so arranged as to pass through
an air cleaning device.

5. A device in accordance with Claim 4, c h a r a c t e r - i z e d in that the air supply is variable in intensity, for instance with an increased supply of air being provided for an increase in effort.

6. A device in accordance with Claim 4, c h a r a c t e r - i z e d in that the air passes through an air cooling device.

7. A device in accordance with Claim 4, c h a r a c t e r - i z e d in that oxygen is added to the air supply.

8. A device in accordance with Claim 7, c h a r a c t e r - i z e d in that the addition of oxygen to the air supply may be regulated irrespective of the effort.

0028209

Fig. 1

Fig. 2

Fig. 3

| | | Application number |
|---|---|---|
| European Patent Office | **EUROPEAN SEARCH REPORT** | EP 80 85 0149 |

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| X | <u>US - A - 3 675 640</u> (J. GATTS) <br> * Figures 2,3; column 1, line 62 - column 2, line 22; column 3, lines 3-22, 47-64; column 4, line 70 - column 5, line 7; column 6, lines 70-73; column 7, lines 23-54 * <br><br> -- | 1,3 | A 63 B 21/00 <br> A 61 B  5/10 |
| X | <u>DE - A - 2 630 293</u> (KEIPER GMBH) <br><br> -- | 1 | |
| X | <u>WO - A - 80/00124</u> (R. POLHEMUS) <br> * Figure 3; page 14, line 6 - page 15, line 33 * <br><br> -- | 1 | **TECHNICAL FIELDS SEARCHED (Int. Cl.³)** <br><br> A 63 B <br> A 61 B |
| | <u>FR - A - 2 323 992</u> (KEIPER GMBH) <br> * The figures; page 1, line 23 - page 2, line 18; page 5, line 6 - page 6, line 32 * <br> & GB - A - 1 516 564 <br><br> -- | 1 | |
| | <u>FR - A - 1 565 617</u> (P. TRONVILLE) <br> * Page 1, right-hand column, paragraph 5 * <br><br> -- | 2,7,8 | **CATEGORY OF CITED DOCUMENTS** <br> X: particularly relevant <br> A: technological background <br> O: non-written disclosure <br> P: intermediate document |
| | <u>FR - A - 2 256 709</u> (G. BRISARD) <br> * The figure; page 1, lines 19-28; page 3, lines 3-7 * <br><br> -- | 1,4,5 | T: theory or principle underlying the invention <br> E: conflicting application <br> D: document cited in the application |
| | <u>FR - A - 2 136 105</u> (E. JEAGER) <br> * Page 3, lines 10-20; page 5, ./. | 1,3 | L: citation for other reasons |
| | | | &: member of the same patent family, corresponding document |

X The present search report has been drawn up for all claims

| Place of search | Date of completion of the search | Examiner |
|---|---|---|
| The Hague | 03-02-1981 | VEREECKE |

# EUROPEAN SEARCH REPORT

**European Patent Office**

## DOCUMENTS CONSIDERED TO BE RELEVANT

| Category | Citation of document with indication, where appropriate, of relevant passages | Relevant to claim |
|---|---|---|
| | line 7 - page 6, line 11 *<br>& GB - A - 1 385 382 | |
| | GB - A - 1 406 342 (LIFECYCLE INC)<br>* Figures 3-6; page 2, line 119 - page 3, line 82; page 4, lines 33-63; page 5, lines 64-70 * | 1-4 |
| | DE - A - 2 436 748 (L. FORSMAN)<br>* Figure 7; page 11, line 17 - page 12, line 4 *<br>& GB - A - 1 436 495 | 1,3 |
| | US - A - 3 395 698 (L. MOREHOUSE)<br>* The figures; column 7, lines 33-61 * | 1,3 |
| L | US - A - 4 184 678 (E. FLAVELL) | 1 |

**CLASSIFICATION OF THE APPLICATION (Int. Cl. 3)**

**TECHNICAL FIELDS SEARCHED (Int. Cl. 3)**

EPO Form 1503.2   06.78